(19) Europäisches Patentamt
European Patent Office
Office européen des brevets

(11) **EP 2 814 450 B1**

(12) **EUROPÄISCHE PATENTSCHRIFT**

(45) Veröffentlichungstag und Bekanntmachung des Hinweises auf die Patenterteilung:
**19.06.2019   Patentblatt 2019/25**

(21) Anmeldenummer: **13703437.7**

(22) Anmeldetag: **12.02.2013**

(51) Int Cl.:
*A61K 8/06* (2006.01)     *A61K 8/37* (2006.01)
*A61K 8/92* (2006.01)     *A61Q 19/00* (2006.01)

(86) Internationale Anmeldenummer:
**PCT/EP2013/052731**

(87) Internationale Veröffentlichungsnummer:
**WO 2013/120823 (22.08.2013 Gazette 2013/34)**

(54) **STABILE WASSER IN ÖL EMULSIONEN MIT HLB-ÄHNLICHEN EMULGATOREN**

STABLE WATER-IN-OIL EMULSIONS WITH HLB-TYPE EMULSIFIERS

ÉMULSIONS EAU DANS L'HUILE STABLES CONTENANT DES ÉMULSIFIANTS DE TYPE HLB

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.02.2012   DE 102012002950**

(43) Veröffentlichungstag der Anmeldung:
**24.12.2014   Patentblatt 2014/52**

(73) Patentinhaber: **Beiersdorf Aktiengesellschaft 20253 Hamburg (DE)**

(72) Erfinder:
• **VON DER FECHT, Stephanie**
  **22880 Wedel (DE)**
• **MÖLLGAARD, Svenja, Lena**
  **22337 Hamburg (DE)**
• **BALCKE, Isabel**
  **22337 Hamburg (DE)**
• **KOCH, Petra**
  **22457 Hamburg (DE)**

(74) Vertreter: **Hartmann, Jost**
  **Beiersdorf AG**
  **Unnastrasse 48**
  **20253 Hamburg (DE)**

(56) Entgegenhaltungen:
EP-A2- 2 123 257          WO-A1-2010/125541
DE-A1-102006 053 360      FR-A1- 2 927 535

• **DATABASE GNPD [Online] MINTEL; 31. Januar 2012 (2012-01-31), Migros: "Zoe - Cream & Oil Body Milk", XP002716995, Database accession no. 1709361**
• **DATABASE GNPD [Online] MINTEL; 30. November 2010 (2010-11-30), Mibelle: "I Am Body - Regenerative Body Milk", XP002716996, Database accession no. 1428287**
• **MEYER J ET AL: "A novel PEG-free emulsifier designed for formulating W/O lotions with a light skin feel", SOFW-JOURNAL SEIFEN, OELE, FETTE, WACHSE, VERLAG FUR CHEMISCHE INDUSTRIE, AUGSBURG, DE, Bd. 131, Nr. 11, 1. Januar 2005 (2005-01-01), Seiten 20-28, XP009104229, ISSN: 0942-7694**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung umfasst kosmetische und/oder dermatologische Zubereitung auf Basis einer Wasser in Öl Emulsion umfassend zwei W/O-Emulgatoren, die sich in ihrem HLB-Wert um maximal 0,5 unterscheiden.

Als kosmetische oder medizinische Zubereitungen sind oftmals Emulsionen, insbesondere W/O-, O/W-, O/W/O oder W/O/W-Emulsionen, im Einsatz. Unter Emulsionen versteht man im Allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten (W/O) in Form feinster Tröpfchen in der anderen Flüssigkeit dispergiert. Die Flüssigkeiten (rein oder als Lösungen) liegen in einer Emulsion in einer mehr oder weniger feinen Verteilung vor, die im Allgemeinen nur begrenzt stabil ist.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter, zum Beispiel elektrische Leitfähigkeit, Sensorik, Anfärbbarkeit der kontinuierlichen Phase, einer O/W-Emulsion ist durch das Wasser geprägt. Bei einer Wasser-in-Öl-Emulsion (W/O-Emulsion, z. B. Butter) handelt es sich um das umgekehrte Prinzip, wobei der Grundcharakter hier durch das Öl bestimmt wird.

Der Stand der Technik kennt mehrere wesentliche Faktoren, die einen positiven Einfluss auf die Stabilität und Rheologie von Emulsionen haben.

Emulsionen benötigen zu ihrer Bildung und zur Stabilisierung im Allgemeinen einen oder mehrere Emulgatoren, Verdicker und/oder Konsistenzgeber, um über einen kosmetisch akzeptablen Zeitraum stabil zu sein, im Allgemeinen 1 Jahr nach dem Öffnen einer kosmetischen Zubereitung.

Eine besondere Herausforderung stellt die Formulierung von fließfähigen Emulsionen dar. Diese werden aufgrund ihrer ansprechenden Verteilbarkeit vom Verbraucher sehr geschätzt, jedoch ist die stabile Formulierung eine technologische Herausforderung.

Zur Stabilisierung von Emulsionen werden häufig ethoxilierte Emulgatoren verwendet, die, allgemein bekannt, zu belastbaren, stabilen Emulsionszubereitungen führen und oft einen relativ breiten sensorischen Bereich abdecken können. Bekannt ist jedoch, dass ethoxilierte Emulgatoren durch ihre PEG-Einheiten als Penetrationsenhancer wirken.

So offenbart EP 1 192 935 A2 W/O-Emulsionen umfassend Polyether wie PEG-45 (dodecyl glycol) copolymer und PEG-22 (dodecyl glycol) copolymer.

Die Verwendung von Polyethylenglykolen und/oder Polyethylenglykolderivaten und deren Abkömmlingen werden in der Öffentlichkeit kontrovers diskutiert, da sie im Verdacht stehen nach topischer Applikation die Haut durchlässiger für Fremdkörper wie z.B. Schadstoffe zu machen.

Weiterhin können unter Einwirkung von Sonnenstrahlung die photoinstabilen Polyethylenglykolhaltigen (PEG)-Emulgatoren zersetzt werden und unschöne Hautreaktionen auslösen.

Aus den genannten Gründen werden vom Verbraucher zunehmend kosmetische Formulierungen gesucht, die frei von dieser Substanzklasse sind.

Um W/O-Emulsionen sensorisch ansprechend entwickeln zu können, werden derzeit dazu aber auch meist PEG-Emulgatoren verwendet. Da viele Verbraucher Produkte suchen, die frei von dieser Substanz-Klasse sind, ist es eine technologische Herausforderung W/O-Emulsionen ohne diese Stoffe zu stabilisieren und gleichzeitig ein ansprechendes Hautgefühl zu erlangen.

Hinsichtlich der Stabilität ist die Herausforderung insbesondere das scaling-up und beim Hautgefühl sind Parameter wie "schnelles und leichtes Einziehen, geringe Klebrigkeit" schwer ohne PEG-Stabilisatoren zu erreichen

Wünschenswert ist es daher Emulsionszubereitung ohne ethoxilierte Emulgatoren zur Verfügung zu stellen, die aber dennoch möglichst breit variierbar und vor allem stabile Emulsionen darstellen.

Weiterhin müssen kosmetische oder dermatologische Zubereitungen einigen ästhetischen und sensorischen Gesichtspunkten genügen um eine ausreichende Verbraucherakzeptanz zu erlangen.

Im Markt ist eine kosmetische Zubereitung "Bebe Zartpflege Zartcreme" mit den Inhaltsstoffen Methylglucoseisostearat, hydrogenated castor oil und Diisotearyl polyglyceryl-3 Dimer Dilinoleat.

DE 60 2004 013 358 T2 offenbart mehrphasige Emulsionen. Bei den beschriebenen Emulsionen handelt es sich um "Mehrfach-Wasser-in-Öl-in-Wasser-Emulsionen" d.h. W/O/W-Systeme. Darüber hinaus wird als W/O-Emulgator Polyglyceryl-3 Diisostearat genannt.

DE 10 2008 028 822 A1 offenbart ein umfangreiches Naschlagewerk zu Emulgatoren und Ölen. Konkret werden kosmetische Stiftzusammensetzungen in Form einer Öl in Wasser Dispersion/Emulsion beschrieben.

DE 199 24 277 A1 beschreibt W/O-Emulsionen und ihre bekannten Vor- und Nachteile. Die Nachteile sollen durch den Einsatz von grenzflächenaktiven Substanzen aus der Gruppe der Alkylmethiconcoplyole beseitigt werden.

US 20030103921 A1 offenbart Antitranspiranthaltige Mikroemulsionen.

Die DE 2830492 A1 und US 20090285876 A1 offenbaren keine W/O-Emulsionen, die mit Hilfe von zwei W/O-Emulgatoren stabilisiert werden.

WO 2009/080657 A2 offenbart W/O-Emulsionen umfassend neben hydrophob modifizierten Polysachariden,

Stärken und/oder Agar Polyglycerol-4 Diisostearat Polyhydroxystearat Sebacat (Isolan GPS ®).

FR 2927535 A2 offenbart stabile Wasser in Öl Emulsionen als kosmetische Zubereitungen umfassend Ester der Fettsäuren und Polyole. Als bevorzugter Ester ist u.a. Polyglycerol-4 Diisostearat Polyhydroxystearat Sebacat (Isolan GPS ®) genannt.

WO 2008/055692 A2 beschreibt silikonfreie Hautschutzmittel umfassend neben Ölen und Polyolen u.a. als Emulgatoren Polyglycerol-4 Diisostearat Polyhydroxystearat Sebacate (GPS) und/oder Polyglyceryl-2 Dipolyhydroxystearat (PGPH). Die Verwendung dieser Emulgatorkombination führt entweder zu nicht-fießfähigen, dafür aber stabilen Formulierungen oder zu kosmetischen Zubereitungen, die bei der Lagerung bei 40 Grad Celcius nach einiger Zeit eine Ölabscheidung zeigen. Darüberhinaus zeichnen sich die mittels Polyglycerol-4 Diisostearat Polyhydroxystearat Sebacate und/oder Polyglyceryl-2 Dipolyhydroxystearat stabilisierten Emulsionen durch eine klebrige, ölige Sensorik aus. Werden leicht speitende Öle zur Optimierung dieser unerwünschten Sensorik eingesetzt, so kommt es ebenfalls zu einem Stabilitätsverlust bei einer längerfristigen Lagerung bei 40°C.

Die Erfindung ist eine kosmetische und/oder dermatologische Zubereitung auf Basis einer Wasser in Öl Emulsion umfassend zwei W/O-Emulgatoren. Die zwei W/O-Emulgatoren unterscheiden sich in ihrem HLB-Wert um maximal 0,5. Bevorzugt ist die Kombination von Emulgatoren mit demselben HLB-Wert. Besonders bevorzugt ist die Verwendung von ausschließlich zwei Emulgatoren.

Als bevorzugte W/O-Emulgatoren werden Diisostearoyl Polyglyceryl-3 Dimer Dilinoleat und Polyglyceryl-4 Diisostearte/Polyhydroxystearate/Sebacate gewählt.

Diisostearoyl Polyglyceryl-3 Dimer Dilinoleate sind als PEG-freie Emulgatoren als Isolan® PDI und Polyglyceryl-4 Diisostearate/Polyhydroxystearate/Sebacate als Isolan® GPS der Fa. Evonik mit einem HLB-Wert von ca. 5 bekannt Überraschender Weise ist es durch die Kombination zweier erfindungsgemäßer W/O-Emulgatoren gelungen, die genannten technischen Herausforderungen zu lösen.

In gleicher Weise kann erfindungsgemäß auf den Zusatz an Polyethylenglykolen und/oder Polyethylenglykolderivaten verzichtet werden. Der Anteil an PEGs liegt daher unter 1 Gew.%, insbesondere 0 Gew.%, bezogen auf die Gesamtmasse der Zubereitung.

Es sind erfindungsgemäß keine weiteren W/O-Emulgatoren zur Stabilisierung notwendig. Hydrophile Stabilisatoren wie Verdicker und Fettalkohole können erfindungsgemäß optional enthalten sein.

Der Anteil an den erfindungsgemäßen W/O-Emulgatoren liegt im Bereich von 1,5 bis 3 Gew.%, bevorzugt 2 bis 2,6 Gew.%, bezogen auf die Gesamtmasse der Zubereitung.

Der Anteil an Polyglyceryl-4 Diisostearate/Polyhydroxystearate/Sebacate wird vorteilhaft im Bereich von 0,1 bis 2,5 Gew.%, bevorzugt 0,3 bis 1,5 Gew.%, besonders bevorzugt 0,5 bis 1 Gew.% und der Anteil an Diisostearoyl Polyglyceryl-3 Dimer Dilinoleate im Bereich 0,1 bis 3 Gew.%, bevorzugt 0,5 bis 2 Gew.%, besonders bevorzugt 1 bis 1,8 Gew.%, bezogen auf die Gesamtmasse der Zubereitung, gewählt.

Die erfindungsgemäßen Zubereitungen umfassen vorteilhaft mindestens ein Hautbefeuchtungsmittel zu einem Anteil von insgesamt 5 bis 20 Gew.%, bezogen auf die Gesamtmasse der Zubereitung. Bevorzugt ist ein Glycerin-Gehalt von 7 bis 15 Gew.% beonders bevorzugt von 10 Gew.%.

Durch den Einsatz der erfindungsgemäßen Emulgatorkombination ist es gelungen, eine W/O-Emulsion zu formulieren, die trotz hoher Mengen an Hautbefeuchtungsmitteln, wie Glycerin, ein sehr ansprechendes Hautgefühl aufweist.

Des Weiteren sind die erfindungsgemäßen Zubereitungen bei Streßtests, wie z.B. Lagerung bei erhöhten bzw. wechselnden Temperturen, stabil. Diese Stabilität besteht nicht nur bei den im Labormaßstab hergestellten 1 bis 5 kg-Ansätzen sondern auch bei größeren Mengen wie z.B. 100 bis 500 kg und auch bei der Produktion im Tonnen-Maßstab. Dieser als "up-scaling" bekannte Prozess ist bekanntermaßen für übliche W/O-Emulsionen besonders sensibel. Für die im Rahmen dieser Erfindung dargestellten Zubereitungen stellte sich der up-scaling-Prozess als überraschend problemlos dar, was sich auf die erfindungsgemäße Emulgatorwahl gründet.

Emulgatoren bewirken, dass zwei nicht miteinander mischbare Flüssigkeiten (zum Beispiel Öl in Wasser) sich zu einer Emulsion vermengen können. Aufgrund des amphiphilen Charakters dringen sie mit ihrem fettlöslichen Teil in das Öl ein. Durch den hydrophilen Teil kann das nun durch Rühren entstandene Öltröpfchen in der wässrigen Umgebung dispergiert werden. Emulgatoren haben primär keinen waschaktiven, tensidischen Charakter. Emulgatoren setzen die Grenzflächenspannung zwischen den beiden Phasen herab und erreichen neben der Verringerung der Grenzflächenarbeit auch eine Stabilisierung der gebildeten Emulsion. Sie stabilisieren die gebildete Emulsion durch Grenzflächenfilme sowie durch Ausbildung sterischer oder elektrischer Barrieren, wodurch das Zusammenfließen (Koaleszenz) der emulgierten Teilchen verhindert wird.

Damit Verbindungen als Emulgatoren wirksam sein können, müssen sie eine bestimmte Molekülstruktur aufweisen. Strukturelles Kennzeichen solcher Verbindungen ist ihr amphiphiler Molekülaufbau. Das Molekül einer solchen Verbindung besitzt wenigstens eine Gruppe mit Affinität zu Substanzen starker Polarität (polare Gruppe) und wenigstens eine Gruppe mit Affinität zu unpolaren Substanzen (apolare Gruppe).

Es wird dabei unterschieden zwischen nicht-ionisch, anionisch und kationischen Emulgatoren. Ein Kennzeichen für die Hydrophilie eines gegebenen Emulgators ist dessen HLB-Wert, der sich nach folgender Formel ergibt: HLB = 20 x (1- $M_{lipophil}$/M), wobei $M_{lipophil}$ für die Molmasse des lipophilen Anteils im Emulgator und M für die Molmasse des gesamten Emulgators steht.

Im Allgemeinen gelten Emulgatoren mit einem HLB-Wert bis ca. 8 als W/O-Emulgatoren. O/W-Emulgatoren hingegen weisen HLB-Werte von größer 8 bis 15 auf. Substanzen mit HLB-Werten größer 15 werden häufig als Lösungsvermittler bezeichnet.

Erfindungsgemäß hat sich nun herausgestellt, dass bei Wahl zweier W/O-Emulgatoren, die beide einen HLB-Wert < 8 aufweisen und beide ähnlichen HLB-Wert aufweisen, es möglich ist kosmetische Zubereitungen zu formulieren, die fließfähig und in ihrer sensorischen Anmutung sehr ansprechend, d.h. nicht klebrig, nicht ölig sind und ein weiches Hautgefühl nach Verteilung verursachen und trotzdem eine gute Stabilität auch bei Lagerung bei verschiedenen Temperaturen aufweisen. Besonders überraschend ist der problemlose up-scaling-Prozess.

Die Kombination zweier W/O-Emulgatoren, die sich in ihrem HLB-Wert um maximal 0,5 unterscheiden, lässt sich somit zur Herstellung von kosmetischen Wasser in Öl Emulsionen mit verbesserter Stabilität und/oder Sensorik verwenden.

In Vergleichsversuchen wurde die Stabilität und Sensorik erfindungsgemäßer Zubereitungen mit denen des Standes der Technik (WO 2008/055692 A2, siehe Tabelle 1) untersucht.

Tabelle 1: Stabilität und Sensorik W/O Emulsionen

| Versuchsnummer | Emulgator | Stabilität | Sensorik |
|---|---|---|---|
| 18 | 1,5 % PGPH | (v) Ölabscheidung | Rückstand ölig, klebriger |
| 19 | 2 % PGPH | v | leichte Sensorik, Rückstand etwas öliger, glänzender |
| 20 | 2,5 % PGPH | Ölabscheidung | |
| 21 | 3 % PGPH | Ölabscheidung | kaum und matter Rückstand, sehr angenehm, nicht ölig, klebriger als Original |

Die Zubereitungen mit nur einem W/O Emulgator mit einem HLB-Wert von 5 (PGPH - Polyglyceryl-2 Dipolyhydroxystearat) zeigen ungenügende Stabilitäten (Ölabscheidungen) und/oder unangenehme Sensorik, wie in obiger Tabelle 1 dargestellt.

Die erfindungsgemäßen Zubereitungen hingegen, mit zwei W/O Emulgatoren, die sich lediglich in ihrem HLB Wert um maximal 0,5 unterscheiden, führen zu einer sensorisch akzeptablen Zubereitung als auch zu stabilen W/O Emulsionen (Beispiel 4).

Tabelle 2: Stabilität und Sensorik erfindungsgemäßer Zubereitung im Vergleich mit Zubereitungen ohne erfindungsgemäßer Emulgatorkombination.

| | Viskosität in mPas | Stabilität unter wechselnden Temperaturen (-12 bis + 60°C) | Sensorik |
|---|---|---|---|
| Bsp. Rezeptur 4 | 5400 | in Ordnung | leicht verteilbar mit angenehmen, pflegenden Rückstand |
| Rezeptur 4 ohne PDI, dafür mit 2,2% GPS | 3700 | in Ordnung | beim Verteilen glitschig, öliger als Bsp. 4 |
| Rezeptur 4 ohne PDI und ohne GPS, dafür mit 2,2% PEG-40 Sorbitan Perisostearate | 3200 | 2 mm Ölabscheidung | beim Verteilen sehr leicht, sehr ölig |
| PDI = Diisostearoyl Polyglyceryl-3 Dimer Dilinoleat GPS = Polyglyceryl-4 Diisostearate/Polyhydroxystearate/Sebacat | | | |

Es zeigte sich, dass die Kombination zweier W/O-Emulgatoren, die sich lediglich in ihrem HLB-Wert um maximal 0,5 unterscheiden zu Zubereitungen führen, die sowohl stabil als auch sensorisch ansprechender sind als ohne die Kombination.

Die Untersuchungen zeigen auch das lediglich ein Emulgator nicht ausreichend ist für das Erreichen der gewünschten Eigenschaften, wie verbesserte Sensorik und Stabilität.

Zur Messung der Viskosität kosmetischer Zubereitungen stehen u.a. Rotationsrheometer zur Verfügung.

Die Messung der Viskosität mit einem Rotationsrheometer erfolgt indem ein Messkörper in der zu vermessenden Substanz rotiert. Die Viskosität der Substanz verhält sich proportional zu der Kraft, mit der sie der Rotation des Messkörpers entgegenwirkt. Dies gilt unter der Voraussetzung, dass Messkörpergeometrie und Drehzahl gleich bleiben. Es gibt dazu sehr unterschiedliche Messkörpergeometrien

- koaxiale Zylinder gemäß DIN 53 019
- Kegel - Platte gemäß DIN 53 019
- Ankerrührer oder T- Spindeln oder
- Tellerförmige Messkörper gemäß ISO 2555

Die Viskosität $\eta$ ist definiert als das Verhältnis aus Schubspannung T und $\dot{\gamma}$ Schergeschwindigkeit

$$\eta = \frac{\tau}{\dot{\gamma}}$$

Die Viskosität verändert sich mit der Schergeschwindigkeit. Damit ist bei der Verarbeitung, Abfüllung oder Anwendung der Produkte darauf zu achten, dass sich das Produkt z.B. beim Pumpen deutlich anders verhalten kann als beim Ausgießen.

Die Viskosität ist also jeweils in dem Schergeschwindigkeitsbereich zu ermitteln, in der die Anwendung liegt, d. h. es müssen verschiedene rheologische Untersuchungsmethoden verwendet werden, wenn man feststellen will, ob die Oberfläche der Creme im Behälter schnell verläuft, ob die Creme angenehm aufzutragen ist und in die Haut einzieht oder ob man berechnen möchte, welche Pumpleistung benötigt wird, um sie durch ein Rohrleitungssystem zu fördern.

Zweckmäßig und im Sinne der vorliegenden Erfindung wird, sofern nicht anders angegeben, die Viskosität $\eta$ mit dem Gerät "Viscotester VT-02" der Firma Haake bei der Temperatur T = 25°C und der Schergeschwindigkeit 10 s$^{-1}$ gemessen.

Überraschend war es darüber hinaus, dass erfindungsgemäß eine sensorisch gleichwertige bzw. verbesserte Zubereitung entwickelt werden konnte, die PEG-frei ist, mehr als doppelt so viel Glycerin enthält und gut im upscaling ist.

Die Erfindung ist daher vorteilhaft eine kosmetische oder dermatologische Zubereitung auf Basis einer Wasser in Öl Emulsion und sie weist insbesondere keine (0%) ethoxilierte Emulgatoren, Polyethylenglykole und/oder Polyethylenglykolderivate auf.

Die erfindungsgemäßen Zubereitungen umfassen vorteilhaft mindestens 10 bis 30 Gew.%, bevorzugt 15 bis 25 Gew.%, besonders bevorzugt 18 bis 22 Gew.% Lipide.

Der Lipid- oder Ölphase werden die Emulgatoren nicht hinzugezählt.

Vorteilhaft kann sich die Ölphase aus bei Raumtemperatur (RT) festen bzw. halbfesten Rohstoffen und flüssigen Rohstoffen zusammensetzen. Der Anteil an festen bzw. halbfesten Bestandteilen bezogen auf die Ölphase beträgt vorteilhaft ca. 40% bis 0,1%, bevorzugt ca. 30 bis 3 % und besonders bevorzugt ca. 15 bis 5 Gew.%, bezogen auf die Gesamtmasse der Ölphase.

Bevorzugte bei RT flüssige Lipidkomponenten werden gewählt aus der Gruppe Paraffinum Liquidum, Isopropyl Palmitate, C13-16 Isoparaffin und natürliche Öle wie Arganöl, Olivenöl, Sonnenblumenöl oder Mandelöl.

Bevorzugt bei Raumtemperatur (RT) feste bzw. halbfeste Lipidkomponenten werden gewählt aus der Gruppe Cera Microcristallina, Cetyl Palmitate und/oder Vaseline (Cera Microcristallina + Paraffinum Liquidum).

Die erfindungsgemäße kosmetische und/oder dermatologische Zubereitung ist eine bei Raumtemperatur fließfähige Wasser in Öl Emulsion und keine Mehrfachemulsion, wie W/O/W- oder O/W/O-Emulsion. Ebenso ist die erfindungsgemäße Emulsion vorteilhaft keine Mikro- oder Nanoemulsion.

Die erfindungsgemäßen Zubereitungen sind vorteilhaft bei Raumtemperatur (20°C) fließfähig.

Weiterhin umfassen die erfindungsgemäßen Zubereitungen ein oder mehrere Puderrohstoffe, die bevorzugt zu einem Anteil von bis zu 5 Gew.%, bevorzugt 0,2 bis 2 Gew.%, bezogen auf die Gesamtmasse der Zubereitung enthalten sind. Bevorzugte Puderrohstoffe sind Aluminum Starch Octenylsuccinat und/oder Talkum.

Die kosmetischen oder dermatologischen Zubereitungen gemäß der Erfindung können ferner kosmetische Hilfsstoffe und weitere Wirkstoffe enthalten, wie sie üblicherweise in solchen Zubereitungen verwendet werden, z. B. Konservierungsmittel, Konservierungshelfer, Bakterizide, Substanzen zum Verhindern des Schäumens, Farbstoffe und Farbpigmente, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate, Selbstbräuner, Puffer, pH Regulatoren, Pflanzliche Extrakte, Tenside, Treibgase, Puder, Sebumabsorbierende Substanzen, UV-Filter, Wirkstoffe wie zum Beispiel Anti Age, Anti-Cellulite, Anti Akne, Anti-Rosacea, Anti-Neurodermitis, Antioxidantien, Moisturiser, Chelatbildner, Antitranspirantien, Bleich- und Färbemittel etc, sofern der Zusatz die geforderten Eigenschaften hinsichtlich PEG-freiheit, Emulgatorgehalt, geforderter Stabilität und Sensorik nicht behindert.

Der Wassergehalt der erfindungsgemäßen Zubereitungen liegt vorteilhaft zwischen 40 und 80 Gew.%, bevorzugt zwischen 50 Gew.% und 70 Gew.%, besonders bevorzugt zwischen 55 Gew.% und 65 Gew.%, jeweils bezogen auf die Gesamtmasse der Zubereitungen.

Bei Einschränkungen auf bevorzugt genannte Stoffe, seien es Lipide, die W/O Emulgatoren oder weitere bevorzugt gennannte Bestandteile, so beziehen sich deren bevorzugten Anteilsbereiche dann auch auf die dann ausgewählten Einzelbestandteile. Die anderen, die durch die Einschränkung ausgeschlossenen Bestandteile, zählen dann nicht mehr zu den aufgeführten Anteilsbereichen hinzu.

Die nachfolgenden Beispiele illustrieren die erfindungsgemäßen Zubereitungen. Die Zahlenangaben beziehen sich auf die Gewichtsanteile in Bezug zur Gesamtmasse der Zubereitung, sofern nichts anderes angegeben ist.

Beispiel 1:

0,5 % Polyglyceryl-4 Diisostearate/Polyhydroxystearate/Sebacate
1,9 % Diisostearoyl Polyglyceryl-3 Dimer Dilinoleate
3% Cera Microcristallina
10% Caprylic/Capric Triglyceride
10% Dicapylyl Ether
1% Talkum
12% Glycerin
1% Propylenglycol
0,1% Hexandiol
0,15% Potassium Sorbate
0,1% Citric Acid
0,2% Sodium Citrate
0,4% Parfüm
mit Aqua ad 100%

Beispiel 2:

0,8 % Polyglyceryl-4 Diisostearate/Polyhydroxystearate/Sebacate
1,9 % Diisostearoyl Polyglyceryl-3 Dimer Dilinoleate
2,5 % Cetylpalmitate
0,1% Cera Microcristallina
4% C13-16 Isoparaffin
4% Argania Spinosa Kernel Oil
6,5% Paraffinum Liquidum
6,5% Isopropyl Palmiate
10% Glycerin
2% Tapoca Starch
0,15% Potassium Sorbate
0,2% Sodium Citrate
0,1% Citric Acid
0,15% Parfüm
mit Aqua ad 100%

Beispiel 3:

1,5 % Polyglyceryl-4 Diisostearate/Polyhydroxystearate/Sebacate
1,5 % Diisostearoyl Polyglyceryl-3 Dimer Dilinoleate
2,7% Shea Butter
7,5% Isohexadecane
5, 3% Dibutyl Adipate
6,5% Paraffinum Liquidum
0,1% Sonnenblumenöl
13,75% Glycerin
0,5% Ethylhexylglycerin
1% Aluminium Starch Octenylsuccinate
1% Nylon-12
0,15% Potassium Sorbate
0,2% Sodium Citrate
0,1% Citric Acid
0,3% Parfüm
mit Aqua ad 100%

Beispiel 4:

0,8 % Polyglyceryl-4 Diisostearate/Polyhydroxystearate/Sebacate
1,4 % Diisostearoyl Polyglyceryl-3 Dimer Dilinoleate
1 % Cera Microcristallina + Paraffinum Liquidum
0,5 % Cera Microcristallina
2 % Paraffinum Liquidum
1% Prunus Amygdalus Dulcis Oil
0,5% Cetyl Palmitate
9,5% Isopropylpalmitate
6% C13-16 Isoparaffin
0,5% Aluminium Starch Octenylsuccinate
10% Glycerin
0,15% Potassium Sorbate
0,7% Magnesium Sulfate
0,1% Maris Sal
0,5% Glyceryl Glucoside
0,2% Sodium Citrate
0,1% Citric Acid
0,35% Parfüm
mit Aqua ad 100%

Beispiel 5:

1,4 % Polyglyceryl-4 Diisostearate/Polyhydroxystearate/Sebacate
0,8 % Diisostearoyl Polyglyceryl-3 Dimer Dilinoleate
0,2 % Cera Microcristallina
1% Shea Butter
2,5% Cera Microcristallina + Paraffinum Liquidum
7,5% Caprylic/Capric Triglyceride
12 % Dicaprylyl Ether
0,1% Olivenöl
1% Talkum
0,2% Aluminium Starch Octenylsuccinate
12% Glycerin
0,1% Caprylyl Glycol
0,15% Potassium Sorbate

0,1% Citric Acid
0,2% Sodium Citrate
0,4% Parfüm
mit Aqua ad 100%

Beispiel 6:

1,1 % Polyglyceryl-4 Diisostearate/Polyhydroxystearate/Sebacate
1,2 % Diisostearoyl Polyglyceryl-3 Dimer Dilinoleate
0,2 % Cera Microcristallina
1% Shea Butter
2,5% Cera Microcristallina + Paraffinum Liquidum
7,5% C13-16 Isoparaffin
12 % Dicaprylyl Ether
0,1% Olivenöl
1% Talkum
0,2% Aluminium Starch Octenylsuccinate
12% Glycerin
0,1% Caprylyl Glycol
0,15% Potassium Sorbate
0,1% Citric Acid
0,2% Sodium Citrate
0,4% Parfüm
mit Aqua ad 100%

**Patentansprüche**

1. Kosmetische und/oder dermatologische Zubereitung auf Basis einer Wasser in Öl Emulsion umfassend nur zwei W/O-Emulgatoren **dadurch gekennzeichnet,**
dass sich die W/O-Emulgatoren in ihrem HLB-Wert um maximal 0,5 unterscheiden, dass der Anteil an W/O-Emulgatoren im Bereich von 1,5 bis 3 Gew.%, bevorzugt 2 bis 2,6 Gew.%, bezogen auf die Gesamtmasse der Zubereitung, gewählt wird und
**dass** der Anteil an Polyethylenglykolen und/oder Polyethylenglykolderivaten unter 1 Gew.%, insbesondere 0 Gew.%, bezogen auf die Gesamtmasse der Zubereitung, beträgt und
ein oder mehrere Puderrohstoffe enthalten sind.

2. Zubereitung nach einem der vorstehenden Ansprüche **dadurch gekennzeichnet, dass** die W/O Emulgatoren Diisostearoyl Polyglyceryl-3 Dimer Dilinoleat und Polyglyceryl-4 Diisostearat/Polyhydroxystearat/Sebacat sind.

3. Zubereitung nach Anspruch 2 **dadurch gekennzeichnet, dass** der Anteil an Polyglyceryl-4Diisostearate/Polyhydroxystearate/Sebacate im Bereich von 0,1 bis 2,5 Gew.%, bevorzugt 0,3 bis 1,5 Gew.%, besonders bevorzugt 0,5 bis 1 Gew.% und der Anteil an Diisostearoyl Polyglyceryl-3 Dimer Dilinoleate im Bereich 0,1 bis 3 Gew.%, bevorzugt 0,5 bis 2 Gew.%, besonders bevorzugt 1 bis 1,8 Gew.%, bezogen auf die Gesamtmasse der Zubereitung, gewählt werden.

4. Zubereitung nach einem der vorstehenden Ansprüche umfassend mindestens ein Hautbefeuchtungsmittel zu einem Anteil von insgesamt 5 bis 20 Gew.%, bezogen auf die Gesamtmasse der Zubereitung.

5. Zubereitung nach Anspruch 4 **dadurch gekennzeichnet, dass** als Hautbefeuchtungsmittel Glycerin gewählt wird.

6. Zubereitung nach einem der vorstehenden Ansprüche umfassend mindestens 10 bis 30 Gew.%, bevorzugt 15 bis 25 Gew.%, besonders bevorzugt 18 bis 22 Gew.%, bezogen auf die Gesamtmasse der Zubereitung, ein oder mehrerer Lipide.

7. Zubereitung nach Anspruch 6 **dadurch gekennzeichnet, dass** das oder die Lipide gewählt werden aus der Gruppe Paraffinum Liquidum, Isopropyl Palmitat, C13-16 Isoparaffin, Cera Microcristallina, Cetyl Palmitat, Vaseline (Cera Microcristallina + Paraffinum Liquidum) und/oder natürliche Öle, insbesondere Arganöl, Olivenöl, Sonnenblumenöl und/oder Mandelöl.

8.  Zubereitung nach Anspruch 1 **dadurch gekennzeichnet, dass** der Anteil an Puderrohstoffen bis zu 5 Gew.%, insbesondere 0,2 bis 2 Gew.%, bezogen auf die Gesamtmasse der Zubereitung, beträgt.

9.  Zubereitung nach Anspruch 8 **dadurch gekennzeichnet, dass** als Puderrohstoff Aluminum Starch Octenylsuccinat und/oder Talkum gewählt wird.

10. Zubereitung nach einem der vorstehenden Ansprüche **dadurch gekennzeichnet, dass** der Wasseranteil im Bereich von 40 bis 80 Gew.%, insbesondere zwischen 55 und 65 Gew.%, bezogen auf die Gesamtmasse der Zubereitung, gewählt wird.

## Claims

1.  Cosmetic and/or dermatological preparation based on a water-in-oil emulsion comprising only two W/O emulsifiers, **characterized in that** the W/O emulsifiers differ in their HLB value by at most 0.5, **in that** the fraction of W/O emulsifiers is chosen in the range from 1.5 to 3% by weight, preferably 2 to 2.6% by weight, based on the total mass of the preparation, and **in that** the fraction of polyethylene glycols and/or polyethylene glycol derivatives is less than 1% by weight, in particular 0% by weight, based on the total mass of the preparation, and one or more powder raw materials are present.

2.  Preparation according to one of the preceding claims, **characterized in that** the W/O emulsifiers are diisostearoyl polyglyceryl-3 dimer dilinoleate and polyglyceryl-4 diisostearate/polyhydroxystearate/sebacate.

3.  Preparation according to Claim 2, **characterized in that** the fraction of polyglyceryl-4 diisostearate/polyhydroxystearate/sebacate is chosen in the range from 0.1 to 2.5% by weight, preferably 0.3 to 1.5% by weight, particularly preferably 0.5 to 1% by weight, and the fraction of diisostearoyl polyglyceryl-3 dimer dilinoleate is chosen in the range 0.1 to 3% by weight, preferably 0.5 to 2% by weight, particularly preferably 1 to 1.8% by weight, based on the total mass of the preparation.

4.  Preparation according to one of the preceding claims, comprising at least one skin moisturizer in a fraction of in total 5 to 20% by weight, based on the total mass of the preparation.

5.  Preparation according to Claim 4, **characterized in that** the skin moisturizer chosen is glycerol.

6.  Preparation according to one of the preceding claims, comprising at least 10 to 30% by weight, preferably 15 to 25% by weight, particularly preferably 18 to 22% by weight, based on the total mass of the preparation, of one or more lipids.

7.  Preparation according to Claim 6, **characterized in that** the lipid or the lipids are selected from the group paraffinum liquidum, isopropyl palmitate, C13-16 isoparaffin, cera microcristallina, cetyl palmitate, Vaseline (cera microcrystallina + paraffinum liquidum) and/or natural oils, in particular argan oil, olive oil, sunflower oil and/or almond oil.

8.  Preparation according to Claim 1, **characterized in that** the fraction of powder raw materials is up to 5% by weight, in particular 0.2 to 2% by weight, based on the total mass of the preparation.

9.  Preparation according to Claim 8, **characterized in that** the powder raw material selected is aluminum starch octenylsuccinate and/or talc.

10. Preparation according to one of the preceding claims, **characterized in that** the water fraction is chosen in the range from 40 to 80% by weight, in particular between 55 and 65% by weight, based on the total mass of the preparation.

**Revendications**

**1.** Préparation cosmétique et/ou dermatologique à base d'une émulsion eau dans huile comprenant uniquement deux émulsifiants E/H, **caractérisée en ce que** les émulsifiants E/H diffèrent d'au plus 0,5 au niveau de leur valeur HLB, **en ce que** la proportion d'émulsifiants E/H est choisie dans la plage allant de 1,5 à 3 % en poids, de préférence de 2 à 2,6 % en poids, par rapport à la masse totale de la préparation, et **en ce que** la proportion de polyéthylène glycols et/ou de dérivés de polyéthylène glycols est inférieure à 1 % en poids, notamment 0 % en poids, par rapport à la masse totale de la préparation, et
une ou plusieurs matières premières en poudre sont contenues.

**2.** Préparation selon l'une quelconque des revendications précédentes, **caractérisée en ce que** les émulsifiants E/H sont le diisostéaroyl polyglycéryl-3 dimère dilinoléate et le polyglycéryl-4 diisostéarate/polyhydroxystéarate/sébacate.

**3.** Préparation selon la revendication 2, **caractérisée en ce que** la proportion de polyglycéryl-4 diisostéarate/polyhydroxystéarate/sébacate est choisie dans la plage allant de 0,1 à 2,5 % en poids, de préférence 0,3 à 1,5 % en poids, de manière particulièrement préférée 0,5 à 1 % en poids, et la proportion de diisostéaroyl polyglycéryl-3 dimère dilinoléate est choisie dans la plage allant de 0,1 à 3 % en poids, de préférence 0,5 à 2 % en poids, de manière particulièrement préférée 1 à 1,8 % en poids, par rapport à la masse totale de la préparation.

**4.** Préparation selon l'une quelconque des revendications précédentes, comprenant au moins un agent d'hydratation de la peau en une proportion d'au total 5 à 20 % en poids, par rapport à la masse totale de la préparation.

**5.** Préparation selon la revendication 4, **caractérisée en ce que** la glycérine est choisie en tant qu'agent d'hydratation de la peau.

**6.** Préparation selon l'une quelconque des revendications précédentes, comprenant au moins 10 à 30 % en poids, de préférence 15 à 25 % en poids, de manière particulièrement préférée 18 à 22 % en poids, par rapport à la masse totale de la préparation, d'un ou de plusieurs lipides.

**7.** Préparation selon la revendication 6, **caractérisée en ce que** le ou les lipides sont choisis dans le groupe constitué par la paraffine liquide, le palmitate d'isopropyle, l'isoparaffine en C13-16, la cire microcristalline, le palmitate de cétyle, la vaseline (cire microcristalline + paraffine liquide) et/ou les huiles naturelles, notamment l'huile d'argan, l'huile d'olive, l'huile de tournesol et/ou l'huile d'amande.

**8.** Préparation selon la revendication 1, **caractérisée en ce que** la proportion de matières premières en poudre est de jusqu'à 5 % en poids, notamment de 0,2 à 2 % en poids, par rapport à la masse totale de la préparation.

**9.** Préparation selon la revendication 8, **caractérisée en ce que** de l'octénylsuccinate d'aluminium et d'amidon et/ou du talc sont choisis en tant que matière première en poudre.

**10.** Préparation selon l'une quelconque des revendications précédentes, **caractérisée en ce que** la proportion d'eau est choisie dans la plage allant de 40 à 80 % en poids, notamment comprise entre 55 et 65 % en poids, par rapport à la masse totale de la préparation.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1192935 A2
- DE 602004013358 T2
- DE 102008028822 A1
- DE 19924277 A1
- US 20030103921 A1
- DE 2830492 A1
- US 20090285876 A1
- WO 2009080657 A2
- FR 2927535 A2
- WO 2008055692 A2